(19) Europäisches Patentamt
European Patent Office
Office européen des brevets

(11) Veröffentlichungsnummer: **0 074 591**
**B1**

(12) # EUROPÄISCHE PATENTSCHRIFT

(45) Veröffentlichungstag der Patentschrift: **29.01.86**

(51) Int. Cl.[4]: **C 07 C 41/38,** C 07 C 43/23

(21) Anmeldenummer: **82108169.2**

(22) Anmeldetag: **04.09.82**

(54) **Verfahren zur Isolierung von Dihydroxybenzol-Monoethern aus Reaktionsgemischen.**

(30) Priorität: **16.09.81 DE 3136810**

(43) Veröffentlichungstag der Anmeldung: **23.03.83 Patentblatt 83/12**

(45) Bekanntmachung des Hinweises auf die Patenterteilung: **29.01.86 Patentblatt 86/5**

(84) Benannte Vertragsstaaten: **AT BE CH DE FR GB IT LI NL**

(56) Entgegenhaltungen:
**DE - A - 2 126 150**
**DE - A - 2 845 429**
**DE - C - 809 197**
**US - A - 3 441 616**

**Chemical Abstracts Band 84, Nr. 9, 1. März 1976 Columbus, Ohio USA T. YOSHIMORI et al. "2,2-Dihydroxydiphenyl ether recovery from 2-Chlorophenol hydrolyzate" Seite 469, Spalte 2, Abstract Nr. 58908c**
**Chemical Abstracts Band 79, Nr. 19, 12. November 1973 Columbus, Ohio, USA Y.I. KORENMAN "Extraction of guaiacol from aqueous solutions" Seite 232, Spalte 1, Abstract Nr. 118901d**

(73) Patentinhaber: **BAYER AG, Konzernverwaltung RP Patentabteilung, D-5090 Leverkusen 1 Bayerwerk (DE)**

(72) Erfinder: **Lenthe, Manfred, Dr., Michaelshöhe 40, D-5068 Odenthal (DE)**
Erfinder: **Allenbach, Udo, Dr., Stammheimer Ring 52, D-5000 Köln 80 (DE)**
Erfinder: **Büttner, Gerhard, Dr., Adolf-von-Menzel-Strasse 1, D-5024 Pulheim (DE)**
Erfinder: **Christmann, Karl-Friedrich, Dr., Liebermannstrasse 9, D-4047 Dormagen 1 (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 074 591 B1

**Beschreibung**

Die Anmeldung betrifft ein Verfahren zur selektiven Isolierung von Dihydroxybenzol-Monoethern aus den bei ihrer Herstellung anfallenden Reaktionsgemischen durch Flüssig-Flüssig-Extraktion mit einem geeigneten Extraktionsmittel.

Die Synthese von Monoethern von Dihydroxybenzolen durch Umsetzung von Dihydroxybenzolen mit Alkyl- bzw. Allylhalogeniden in einem Lösungsmittel in Gegenwart einer Base ist bekannt (DE-OS 2 845 429, 2 932 458, US-P 3 474 171).

Bei dieser Synthese sind eine Reihe von Nebenreaktionen möglich, von denen besonders die weitere Veretherung des Monoethers gemäss

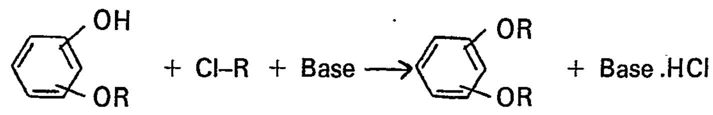

eine erhebliche Ausbeuteverminderung des gewünschten Monoethers verursacht. Dieser Effekt verstärkt sich mit zunehmender Aktivität des eingesetzten Chlorkohlenwasserstoffs.

Die bekannten Verfahren versuchen z.B. durch entsprechende Wahl von Lösungsmitteln zu optimalen Ausbeuten zu gelangen. So sind z.B.Ethanol, Aceton, dipolare aprotische Lösungsmittel oder Polyhydroxyalkylether als besonders geeignete Lösungsmittel für die Herstellung von Monoethern des Brenzkatechins bekannt (vgl. DE-OS 2 932 458).

Da es sich bei der oben erwähnten Bildung des Diethers um eine Folgereaktion handelt, wäre es vorteilhaft, Brenzkatechin nicht vollständig umzusetzen, sondern die Monoverätherung bei einem genau zu bestimmenden Umsatz zu unterbrechen. Durch diesen Eingriff könnte eine hohe Selektivität der Monoetherbildung erzielt und nach Abtrennung und Rückführung des nicht umgesetzten Dihydroxybenzols auch eine wesentlich höhere Ausbeute an gewünschtem Produkt erreicht werden.

Destillative Trennungen der Mono- und Diether sind oft nicht möglich, da die Siedepunkte beider Verbindungen zu nahe beieinander liegen. Ausserdem kann es bei den für eine Destillation erforderlichen Temperaturen zu Folgereaktionen und somit Ausbeuteverlusten kommen.

Aus der DE-OS 2 845 429 ist es weiterhin bekannt, Reaktionsgemische zu extrahieren, die bei der Herstellung von Dihydroxymonoethern erhalten werden.

Aus der DE-OS 2 845 429 ist es auch bekannt, die Reaktion zur Herstellung von Dihydroxymonoethern in aprotischen Lösungsmitteln, wie n-Octan auszuführen.

Gemäss Chemical Abstracts 79, 118 901 d (1973) kann man Guajacol aus wässrigen Lösungen mit einer Reihe von organischen Lösungsmitteln extrahieren.

Das erfindungsgemässe Verfahren zur Isolierung von Dihydroxybenzol-Monoethern aus Reaktionsgemischen in denen neben Lösungsmitteln und Produkten noch nicht umgesetztes Dihydroxybenzol vorliegt wird durch die Literatur weder offenbart noch nahegelegt.

Es wurde nun gefunden, dass man nicht umgesetztes Dihydroxybenzol-Monoether selektiv aus den bei ihrer Herstellung anfallenden Reaktionsgemischen isolieren kann, indem man das Reaktionsgemisch gegebenenfalls in Gegenwart von Wasser mit gesättigten oder ungesättigten, offenkettigen, verzweigten oder cyclischen Kohlenwasserstoffen, die mit dem Reaktionsgemisch nicht oder nicht vollständig mischbar sind, die über 50 °C sieden und mindestens 5 C-Atome/Molekül besitzen, behandelt.

Bei diesem Verfahren wird der Dihydroxybenzol-Monoether zu einem erheblichen Teil von dem Extraktionsmittel aufgenommen, während das Dihydroxybenzol weitestgehend in der Raffinatphase verbleibt und nach geeigneten Aufarbeitungsschritten in die Reaktion zurückgeführt werden kann. Der in der Extraktphase angereicherte bzw. isolierte Monoether kann entweder in der vorliegenden gelösten Form weiterverarbeitet, nach entsprechender Behandlung reextrahiert oder als reine Substanz isoliert werden.

Es war überraschend, dass die Dihydroxybenzolmonoether von den Dihydroxybenzolen durch eine so einfach durchzuführende Massnahme wie die Flüssig-Flüssig-Extraktion getrennt werden können. Dies war deshalb überraschend, weil beide Verbindungstypen in ihrem chemischen und physikalischen Eigenschaften sehr ähnlich sind. So besitzen beide freie OH-Gruppen und haben ähnliche Säurestärken. Beide Verbindungstypen besitzen aber auch sehr ähnliche polare Eigenschaften. Gerade letztere sind für ein Flüssig-Flüssig-Extraktionsverfahren von grosser Bedeutung. Als Mass dafür kann der Azentrizitätsfaktor dienen. Dieser stellt ein Mass für die Komplexität eines Moleküls hinsichtlich seiner Geometrie (Azentrizität, Sphärizität und Polarität) dar (vgl. «The properties of Gases and Liquids», R.C. Reid et al, 3. Aufl., Mac Graw Hill Book Company).

So besitzt Brenzkatechin einen Azentrizitätsfaktor von 0,68879. Isopropoxyphenol besitzt einen Azentrizitätsfaktor von 0,61865. Beide Werte liegen verhältnismässig nahe beieinander.

Eine im wesentlichen unpolare Verbindung wie n-Octan hat demgegenüber einen Faktor von 0,396.

Das erfindungsgemässe Trennungsverfahren lässt sich besonders bevorzugt auf die Gemische von Brenzkatechin mit reinen Monoalkylethern wie Isopropylether, reinen Monoalkenylethern wie Methallylether sowie reinen cyclischen Alkylethern wie 2,2-Dimethyl -7- hydroxycumaran anwenden.

Besonders vorteilhaft lässt sich das erfindungsgemässe Verfahren dann einsetzten, wenn die Monoveretherung in Alkoholen wie Methanol, Ethanol, in Ketonen wie Aceton, in dipolar aprotischen Lösungsmitteln wie Dimethylsulfoxid oder in Polyhydroxyalkylethern wie Glycolmonomethylether sowie deren Gemischen mit Wasser durchgeführt wird.

Besonders geeignete Extraktionsmittel sind gesättigte und ungesättigte Kohlenwasserstoffe sowie deren Gemische mit Siedepunkten zwischen 80 und 300 °C.

Die Auswahl besonders geeigneter Extraktionsmittel für das erfindungsgemässe Verfahren hängt auch von den zu trennenden Gemischen ab.

So sind zur Trennung von Brenzkatechin und Brenzkatechinmonoalkenylethern wie Brenzkatechinmonomethallylether Petroleum (Sdp. 170–250 °C), Dodekan (n und i), Heptan, Pentamethylhepten, Tetraisobutylen besonders geeignet.

Zur Trennung von Brenzkatechin und gesättigten sowie cyclischen Brenzkatechinmonomethylethern wie 2,2-Dimethyl -7- hydroxycumaran sind 4,4-Dimethylpentan, Dodekan (n und i), Tripropylen und Pentamethylhepten besonders geeignet.

Als Extraktionsmittel kommen offenkettige, verzweigte cyclische, gesättigte sowie ungesättigte Kohlenwasserstoffe bzw. Gemische verschiedener Kohlenwasserstoffe in Betracht, deren Siedepunkte über 50 °C liegen bzw. deren Kohlenstoffzahl über 5 pro Molekül beträgt. Nach oben wird die Reihe der verwendungsfähigen Extraktionsmittel durch den Schmelzpunkt begrenzt, der unter der Betriebstemperatur der Extraktion liegen muss.

Bei weiterer Verarbeitung des Extraktes ist darauf zu achten, dass eine physikalische oder chemische Trennung zwischen den extrahierten Substanzen und dem Extraktionsmittel möglich sein muss.

Das Extraktionsergebnis lässt sich in einigen Fällen noch durch das Hinzufügen von Hilfssubstanzen verbessern. So wurde z.B. gefunden, dass ein Wasserzusatz die Selektivität der Extraktion erhöht.

Die Verfahrensbedingungen entsprechen den in der allgemeinen Literatur (z.B. R.H. Perry, C.H. Chilton, Chemical Engineers Handbook, Mac Graw-Hill, New York) beschriebenen Verfahrensformen für die Flüssig-Flüssig-Extraktion.

Weitere Varianten sind denkbar, z.B. kann das Extraktionsmittel bereits in der Reaktion eingesetzt werden, um hier die Funktion einer Schutzphase auszuüben. Bei kontinuierlicher Durchführung in einem geeigneten Extraktionsapparat könnte so die Ausbeute der Veretherung erheblich angehoben werden. Dazu wird während der Reaktion extrahiert und das Extraktionsmittel von Monoether in einer parallel betriebenen Kolonne z.B. destillativ getrennt. Bei diesem Verfahren wird der gebildete Monoether in einer Schutzphase aufgenommen und somit der Reaktion weitestgehend entzogen, wodurch er nicht zum Diether weiterreagieren kann. Ausbeute und Selektivität der Reaktion werden dadurch gesteigert.

Einsatzmöglichkeiten für das erfindungsgemässe Verfahren, bestehen auch in der selektiven Isolierung von hochsiedenden Monoethern, die sich durch Destillation nur unter relativ hohen Ausbeuteverlusten aus den Reaktionsgemischen gewinnen lassen.

So kann z.B. das durch Claisen-Umlagerung und Cyclisierung aus Methallyloxyphenol gewonnene 2,2-Dimethyl -7- hydroxycumaran (cyclischer Dihydroxybenzol-Monoether) aus dem nach der Cyclisierung vorliegenden Gemisch, das als Lösungsmittel z.B. Glykolmonomethylether und nicht umgesetztes Brenzkatechin enthält, extrahiert werden.

**Beispiele**

1. Zur Überprüfung der Extraktionswirkung verschiedener Kohlenwasserstoffe wurde ein Ansatz zur Herstellung von Methallyloxyphenol durch Umsetzung von Brenzkatechin mit Methallylchlorid und Natriumcarbonat als Base in Glykolmonomethylether gemäss folgender Vorschrift durchgeführt:

In einem 2,5 l-Glasreaktor mit aufgesetzter kleiner Kolonne und einem Wasserabscheider werden 1,150 g Glykolmonomethylether, 337 g Brenzkatechin technisch (3 Mol), 178 g (1,68 Mol) wasserfreie Soda und 5 g Natriumdithionit vorgelegt. Man leitet einen schwachen $N_2$-Strom durch die gut gerührte Suspension und heizt auf ca. 110 °C Innentemperatur auf. Über einen Tropftrichter oder eine Dosierpumpe werden im Verlauf von 1 Stunde 407 g (4,5 Mol) Methallylchlorid (MAC) (bei Kreislauffahrweise eine entsprechende Menge an Rückführ-MAC) gleichmässig zudosiert. Bereits beim Aufheizen und während der nachfolgenden Reaktion setzt ein lebhafter $CO_2$-Strom ein. Während der gesamten Reaktionsdauer von 4,5 Std. (einschliesslich Dosierung) wird das gebildete Reaktionswasser weitgehend ausgeschleust. Man kühlt auf 20 °C ab und filtriert das gebildete Kochsalz ab. Den farblosen Filterrückstand wäscht man zweimal mit wenig Lösungsmittel nach und vereinigt Filtrat- und Waschlösungsmittelmengen.

Nach Gaschromatogramm enthält die organische Phase 382 g Brenzkatechin-monomethallylether (Kp = 58 °C bei 0,1 Torr), das sind 78% Ausbeute der Theorie.

Diese Lösung wird zur Simulation eines geringeren Brenzkatechin-Umsatzes mit Brenzkatechin versetzt, so dass das in die Extraktion eingesetzte Gemisch folgende Zusammensetzung besitzt:

| | |
|---|---|
| Brenzkatechin | 10,6 Gew.-% |
| Methallyloxyphenol | 9,5 Gew.-% |
| Diether | 2,3 Gew.-% |
| Glykolmonomethylether | 75,2 Gew.-% |

Diese Lösung wird mit jeweils der gleichen Menge des Extraktionsmittels verrührt. Nach

Trennung der beiden Phasen (Raffinat und Extrakt) werden die gaschromatographischen Gehalte (innerer Standard = Gewichtsanteile) ermittelt. Die Ergebnisse sind in der folgenden Tabelle zusammengestellt.

Es ist festzustellen, dass die erfindungsgemässen Kohlenwasserstoffe eine hohe Selektivität für die Extraktion des Monoethers aus der Polyhydroxybenzolhaltigen Lösung aufweisen.

So lassen sich z.B. bei der Verwendung von n-Dodekan (Extraktionsfaktor = 23,4) in z.B. einer mehrstufigen Gegenstromextraktion Brenzkatechin und Monoether nahezu vollständig voneinander trennen.

Der Effekt bezüglich der Gesamtausbeute der Monoveretherung von z.B. Brenzkatechin liegt in einer Erhöhung der Monoetherausbeute von 78 auf ca. 89%.

Tabelle 1: Ergebnisse der Extraktionsversuche mit Methallyloxyphenol

| Extraktionsmittel | | Gehalte/Gew.-% Extrakt | | Raffinat | | $f_1 = \frac{BC_R}{BC_E}$ | $f_2 = \frac{MOP_R}{MOP_E}$ | $\frac{f_1}{f_2}$ |
|---|---|---|---|---|---|---|---|---|
| | Sdp. °C | $BC_E$ | $MOP_E$ | $BC_R$ | $MOP_R$ | | | |
| <u>Einsatz (Gew.-%):</u> BC: 10,68 / MOP: 9,45 / DE: 2,29 / 3MABC: 0,21 | | | | | | | | |
| Cyclohexan | 81 | 1,04 | 2,56 | 8,04 | 6,20 | 8 | 2 | 3 |
| n-Heptan | 98 | 0,18 | 1,51 | 10,31 | 7,95 | 57 | 5 | 10 |
| 2,4-Dimethylpentan, i-Heptan | 81 | 0,17 | 1,36 | 10,13 | 7,98 | 60 | 6 | 10 |
| i-Oktan | | 0,17 | 1,32 | 9,85 | 8,42 | 58 | 6 | 9 |
| Waschbenzin | 100–140 | 0,33 | 1,84 | 9,92 | 7,49 | 30 | 4 | 7 |
| Ligroin | 30–110 | 0,31 | 1,68 | 9,66 | 7,56 | 31 | 5 | 7 |
| n-Dodekan | 215 | 0,05 | 0,97 | 10,40 | 8,63 | 208 | 9 | 23 |
| i-Dodekan | | 0,08 | 0,99 | 10,55 | 8,58 | 132 | 9 | 15 |
| Terapin | 155–185 | 0,28 | 1,70 | 9,70 | 7,42 | 35 | 4 | 8 |
| Sangajol | 140–200 | 0,26 | 1,65 | 9,68 | 7,50 | 37 | 5 | 8 |
| Petroleum | 170–250 | 0,05 | 1,30 | 10,15 | 7,87 | 203 | 6 | 33 |
| <u>Einsatz (Gew.-%):</u> BC: 10,74 / MOP: 9,21 / DE: 2,17 / 3MABC: 0,12 / 3IBBC: 0,40 | | | | | | | | |
| Diisobutylen (Gemisch) | | 1,21 | 2,75 | 9,23 | 6,53 | 8 | 2 | 3 |
| Tripropylen (Gemisch) | | 0,42 | 1,93 | 10,00 | 7,28 | 24 | 4 | 6 |
| Tetrapropylen (Gemisch) | | 0,22 | 1,31 | 10,58 | 8,05 | 48 | 6 | 8 |
| 2,4,4,6,6-Pentamethyl-1-hepten | | 0,15 | 1,29 | 10,52 | 8,01 | 70 | 6 | 11 |
| Tetraisobutylen (Gemisch) | | 0,10 | 0,92 | 10,85 | 8,43 | 109 | 9 | 12 |

<u>Erläuterungen der Abkürzungen</u>

| | |
|---|---|
| BC | Brenzkatechin |
| MOP | Methallyloxyphenol |
| DE | Brenzkatechindimethallylether |
| 3MABC | 3-Methallylbrenzkatechin |
| 3IBBC | 3-Isobutenylbrenzkatechin |

2. Zur weiteren Untersuchung des erfindungsgemässen Verfahrens wurde ein Gemisch mit folgender Zusammensetzung in die Extraktion eingesetzt:

| Gehalte: | |
|---|---|
| Brenzkatechin | 0,7 Gew.-% |
| 2,2-Dimethyl-7-hydroxy-cumaran (7-OH) | 12,1 Gew.-% |
| Glykolmonomethylether | 83,0 Gew.-% |

Dieses aus einer Cyclisierung von 3-Methallylbrenzkatechin gewonnene Reaktionsgemisch wurde mit jeweils der gleichen Menge Extraktionsmittel vermischt. Nach Phasentrennung wurden die Massen der beiden Phasen sowie die Gehalte bestimmt. Die Ergebnisse dieser Versuche sind in der Tabelle 2 dargestellt.

Es zeigt sich, dass Brenzkatechin nahezu vollständig im Raffinat verbleibt, 7-OH wird bei einmaliger Extraktion jedoch bis zu 26% extrahiert, (n-Dodekan).

Tabelle 2: Ergebnisse der Extraktion von 2,2-dimethyl-7-hydroxycumaran (7-OH)
BC = Brenzkatechin

| Extraktionsmittel | Gehalte/Gew.-% Extrakt $BC_E$ | Extrakt $7\text{-}OH_E$ | Raffinat $BC_R$ | Raffinat $7\text{-}OH_R$ | $f_1 = \frac{BC_R}{BC_E}$ | $f_2 = \frac{7\text{-}OH_R}{7\text{-}OH_E}$ | $\frac{f_1}{f_2}$ |
|---|---|---|---|---|---|---|---|
| Cyclohexan | 0,06 | 2,42 | 0,60 | 8,81 | 10 | 4 | 3 |
| n-Heptan | 0,01 | 0,92 | 0,69 | 11,24 | 69 | 12 | 6 |
| 2,4-Dimethylpentan | – | 0,83 | 0,71 | 11,07 | ∞ | 13 | |
| i-Oktan | 0,01 | 0,31 | 0,72 | 11,07 | 72 | 12 | 6 |
| Waschbenzin | 0,03 | 1,78 | 0,66 | 9,72 | 22 | 6 | 4 |
| Ligroin | 0,01 | 1,31 | 0,70 | 10,88 | 70 | 8 | 8 |
| n-Dodekan | – | 0,48 | 0,72 | 11,79 | ∞ | 25 | ∞ |
| i-Dodekan | – | 0,49 | 0,71 | 11,50 | ∞ | 24 | ∞ |
| Terapin | 0,01 | 1,21 | 0,69 | 10,08 | 69 | 8 | 8 |
| Sanoajol | 0,01 | 1,19 | 0,69 | 10,58 | 69 | 9 | 8 |
| Petroleum | 0,23 | 1,44 | 0,63 | 10,87 | 3 | 8 | 0 |
| Diisobutylen (Gemisch) | 0,10 | 3,12 | 0,70 | 9,97 | 7 | 3 | 2 |
| Tripropylen (Gemisch) | – | 1,88 | 0,73 | 10,00 | ∞ | 5 | ∞ |
| Tetrapropylen (Gemisch) | 0,09 | 1,10 | 0,77 | 10,81 | 9 | 10 | 1 |
| 2,4,4,6,6-Pentamethylhepten | – | 1,11 | 0,76 | 10,92 | ∞ | | ∞ |
| Tetraisobutylen (Gemisch) | | | 0,76 | 11,30 | | 10 | |

## Patentansprüche

1. Verfahren zur Isolierung von Dihydroxybenzol-Monoethern aus Reaktionsgemischen, in denen neben Lösungsmittel und Produkten noch nicht umgesetztes Dihydroxybenzol vorliegt, dadurch gekennzeichnet, dass dieses Reaktionsgemisch mit gesättigten oder ungesättigten, offenkettigen, verzweigten oder cyclischen Kohlenwasserstoffen, die mit dem Reaktionsgemisch nicht oder nicht vollständig mischbar sind, die über 50 °C sieden und mindestens 5 C-Atome/Molekül besitzen, behandelt wird.

2. Verfahren nach Anspruch 1, dadurch gekennzeichnet, dass die Reaktion zur Herstellung des Monoethers in Gegenwart eines Extraktionsmittels durchgeführt wird.

3. Verfahren nach Anspruch 1, dadurch gekennzeichnet, dass zur Erhöhung der Selektivität der Extraktion in Gegenwart von Wasser gearbeitet wird.

## Revendications

1. Procédé pour isoler des monoéthers dihydroxybenzéniques de mélanges réactionneles, dans lesquels se trouve du dihydroxybenzène n'ayant pas encore réagi, à côté du solvant et de produits, caractérisé en ce que ce mélange réactionnel est traité avec des hydrocarbures saturés ou non saturés à chaîne ouverte, ramifiés ou cycliques qui ne sont pas miscibles ou qui ne sont pas entièrement miscibles aux mélanges réactionnels, qui bouillent au-dessus de 50 °C et qui ont au moins 5 atomes de carbone par molécule.

2. Procédé suivant la revendication 1, caractérisé en ce que la réaction pour la production du monoéther est conduite en présence d'un agent d'extraction.

3. Procédé suivant la revendication 1, caractérisé en ce qu'on opère en présence d'eau pour accroître la sélectivité de l'extraction.

## Claims

1. Process for the isolation of dihydroxybenzene monoethers from reaction mixtures in which still unreacted dihydroxybenzene is present in addition to solvent and products, characterised in that this reaction mixture is treated with saturated or unsaturated, open-chain, branched or cyclic hydrocarbons which are not miscible or not completely miscible with the reaction mixture and which boil above 50 °C and have at least 5 C atoms per molecule.

2. Process according to Claim 1, characterised in that the reaction to prepare the monoether is carried out in the presence of an extracting agent.

3. Process according to Claim 1, characterised in that to increase the selectivity of the extraction it is carried out in the presence of water.